# EUROPEAN PATENT APPLICATION

(11) **EP 3 992 143 A1**
(43) Date of publication of application: **04.05.2022**
(21) Application number: 20832976.3
(22) Date of filing: 24.06.2020
(51) Int. Cl.: C01B 3/00, B01J 20/22, C07C 63/15, C07C 65/21, C07C 69/76, C07C 229/38, C07C 229/58

(54) **HYDROGEN STORAGE MATERIAL CONTAINING METAL ORGANIC STRUCTURE**

(30) Priority: 25.06.2019 JP 2019117030
(71) Applicant: Rikkyo Educational Corporation, Tokyo 171-8501 (JP); Nippon Soda Co., Ltd., Tokyo 100-8165 (JP)
(72) Inventor: MINOURA, Mao, Tokyo 171-8501 (JP); SUGAMATA, Koh, Tokyo 171-8501 (JP); YANAGISAWA, Daichi, Tokyo 171-8501 (JP); KOBAYASHI, Sho, Tokyo 171-8501 (JP); IIHAMA, Teruyuki, Tokyo 100-8165 (JP)
(74) Representative: Mewburn Ellis LLP
(86) International application number: PCT/JP2020/024773
(87) International publication number: WO 2020/262452

(57) **Abstract**

The object of the present invention addresses a problem of providing a novel hydrogen storage material containing a metal-organic framework that can effectively store hydrogen. Hydrogen can be effectively stored by use of a hydrogen storage material containing a metal-organic framework, the metal-organic framework comprising a carboxylate ion of formula (I) and a multivalent metal ion, wherein the carboxylate ion and the multivalent metal ion are bound to each other. (In formula (I), X is an unsubstituted or substituted C2-C20 alkyl group, an unsubstituted or substituted alkenyl group, an unsubstituted or substituted alkynyl group, an unsubstituted or substituted alkoxy group, an unsubstituted or substituted alkenyloxy group, an unsubstituted or substituted alkynyloxy group, a benzyloxy group, an unsubstituted or substituted alkylsulfanyl group, an unsubstituted or substituted alkenylsulfanyl group, an unsubstituted or substituted alkynylsulfanyl group, an unsubstituted or substituted alkylamino group, an unsubstituted or substituted dialkylamino group, an unsubstituted or substituted alkenylamino group, an unsubstituted or substituted dialkenylamino group, an unsubstituted or substituted alkynylamino group, an unsubstituted or substituted dialkynylamino group, a phenyl group, a sulfanyl group or an unsubstituted or substituted alkoxycarbonyl group.)

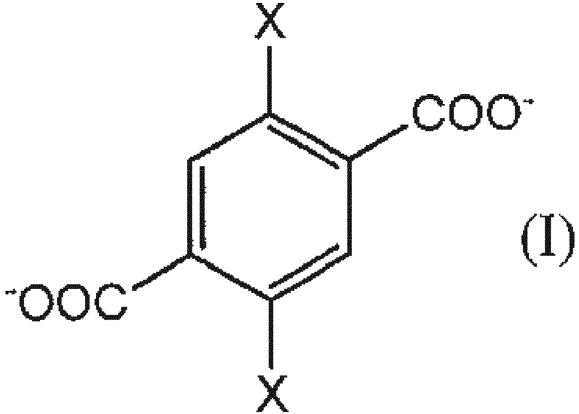

## Description

### Technical Field

The present invention relates to a novel hydrogen storage material containing a metal-organic framework and a hydrogen storage method and a hydrogen storage tank using the hydrogen storage material.

The present application claims priority on the basis of Japanese Patent Application No. 2019-117030 filed on June 25, 2019, the contents of which are hereby incorporated by reference.

### Background Art

A metal-organic framework (hereinafter sometimes referred to as "MOF") is a substance in a solid state that has a macromolecular structure with a space inside (that is, pores) by combining metal ions and a crosslinkable organic ligand that connects them. The metal-organic framework has been of great interest for more than a decade as a porous material with such a function as gas storage or separation.

As the crosslinkable organic ligand, an oxygen donor ligand and a nitrogen donor ligand have been often used.

MOF-5, obtainable by a Solvothermal method in which terephthalic acid is employed as a crosslinkable organic ligand and Zn(NO₃)₂•6H₂O is employed in N,N-dimethylformamide, is known to be able to store 7.1% by mass of hydrogen under conditions of temperature of 77 K and 4 MPa (see Non-patent Documents 1 to 3).

It is also known that an IRMOF (isoreticular Metal-Organic Framewark) isomorphic to MOF-5 can be provided by use of a terephthalic acid derivative having an n-propoxy group and an n-pentoxy group on the positions 2 and 5 (see Non-patent Document 2).

As examples of MOFs using a 2,5-disubstituted terephthalic acid, there are known MOFs obtainable by use of a terephthalic acid having an ω-(carbazol-9-yl)alkoxy group (the alkylene chain has 3, 6 and 8-12 carbon atoms) (see Non-patent Documents 4 and 5), an alkoxy group (6-14 carbon atoms) (see Non-patent Document 6), a methyl group, a methoxy group, a chloro group, an ethyl group and an ethoxy group (see Patent Document 1), a hydroxy group and an acetoxy group (see Non-patent Document 7), an isopropoxy group, an allyloxy group and a propargyloxy group (see Non-patent Document 8), a pyrazol-1-yl group (see Non-patent Document 9), a 4-methylbenzoyl group (see Non-patent Document 10), a benzoyl group (see Non-patent Document 11), a benzyloxy group (see Non-patent Document 12) and a bromo group (see Non-patent Document 13) and a phenyl group (see Non-patent Document 14), on the positions 2 and 5, as an organic ligand.

### Prior Art Documents

### Patent Documents

Patent Document 1: US Patent Application Publication No. 2010-75123

### Non-patent Documents

Non-patent Document 1: H. Li, M. Eddaudi, M. O'Keefe, O. M. Yaghi, Nature, 402, 276 (1999)
Non-patent Document 2: M. Eddaudi, J. Kim, N. Rosi, D. Vodak, J. Wachter, M. O'Keefe, O. M. Yaghi, Science 2002, 295 (5554), 469.
Non-patent Document 3: S. Kaye, A. Daily, O. M. Yaghi, J. Long, J. Am. Chem. Soc. 2007, 129(46), 14176.
Non-patent Document 4: E. Y. Choi, S. H. Lee, O. P. Kwon, Bull. Korean Chem. Soc. 2012, 33(7), 2431.
Non-patent Document 5: E. Y. Choi, S. B. Lee, H. Yun, S. H. Lee, C. Gao, Y. Shin, O. P. Kwon, Bull. Korean Chem. Soc. 2013, 34(12), 3903.
Non-patent Document 6: E. Y. Choi, C. Gao, H. J. Lee, O. P. Kwon, S. H. Lee, Chem. Comunn. 2009, 7563.
Non-patent Document 7: T. Yamada, H. Kitagawa, Supramolecular Chemistry 2011, 23, 315.
Non-patent Document 8: A. Schneemann, E. D. Bloch, S. Henke, P. L. Llewellyn, J. R. Long, R. A. Fischer, Eur. Chem. J. 2015, 21, 18764.
Non-patent Document 9: E. Gao, J. Xing, Y. Qu, X, Qiu, M. Zhu, Appl. Organometal Chem. 2018, 32, e4469
Non-patent Document 10: X. Zheng, S. Q. Guo, X. Y. Yu, J. K. Hu, Y. H. Luo, H. Zhang, X. Chen, Inorganic Chemistry Communication 2012, 18, 29.
Non-patent Document 11: F. Yue, B. Li, X. Zhu, Y. Luo, J. Hu, X. Wang, H. Zhang, Journal of Coordination Chemistry, 2013, 66(2), 243.
Non-patent Document 12: h. Deng, C. J. Doona, H. Furukawa, R. B. Ferreira, J. Towne, C. B. Knobler, B. Wang, O. M. Yaghi, Science 2010, 327, 846.
Non-patent Document 13: J. Gascon, M. D. Hernandez-Alonson, A. R. Almeida, G. P. M. van Klink, F. Kapteijin, G. Mui, ChemSusChem 2008, 1, 981.
Non-patent Document 14: E. R. Engel, A. Jouaiti, C. X. Bezuidenhout, M. W. Hosseini, L. J. Barbour, Angew. Chem. Int. Ed. 2017, 56, 8874.

### Summary of the Invention

### Objects to be Solved by the Invention

As described above, various MOFs are known, but not many MOFs capable of effectively storing hydrogen are not known, in view of the nature of the gas.

An object of the present invention is to provide a novel hydrogen storage material containing a MOF that can effectively store hydrogen.

### Means to Solve the Object

As a result of diligent studies to solve the above problems, the present inventors have found that an MOF to be obtained by use of a combination of a terephthalic acid having a substituent each on the position 2 and the position 5 and various metal salts, even if the MOF is conventionally known, unexpectedly has a capability of storing hydrogen, and have completed the present invention.

That is, the present invention is specified by the following items that follows:
[1] A hydrogen storage material containing a metal-organic framework, the metal-organic framework comprising a carboxylate ion of formula (I) and a multivalent metal ion, wherein the carboxylate ion and the multivalent metal ion are bound to each other: 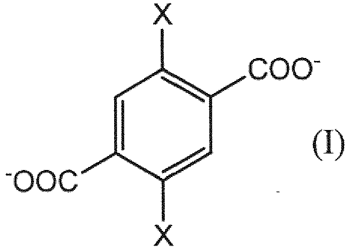 (wherein in formula (I), X is an unsubstituted or substituted C2-C20 alkyl group, an unsubstituted or substituted alkenyl group, an unsubstituted or substituted alkynyl group, an unsubstituted or substituted alkoxy group, an unsubstituted or substituted alkenyloxy group, an unsubstituted or substituted alkynyloxy group, a benzyloxy group, an unsubstituted or substituted alkylsulfanyl group, an unsubstituted or substituted alkenylsulfanyl group, an unsubstituted or substituted alkynylsulfanyl group, an unsubstituted or substituted alkylamino group, an unsubstituted or substituted dialkylamino group, an unsubstituted or substituted alkenylamino group, an unsubstituted or substituted dialkenylamino group, an unsubstituted or substituted alkynylamino group, an unsubstituted or substituted dialkynylamino group, a phenyl group, a sulfanyl group or an unsubstituted or substituted alkoxycarbonyl group).
[2] A hydrogen storage material containing a metal-organic framework, the metal-organic framework comprising a carboxylate ion of formula (I), a multivalent metal ion and an organic ligand containing two or more nitrogen atoms (with the proviso that the carboxylate ion of formula (I) is excluded) or an organic ligand containing a nitrogen atom and a carboxy ionic group (with the proviso that the carboxylate ion of formula (I) is excluded), wherein the carboxylate ion of formula (I) and the organic ligand are bound to the multivalent metal ion: 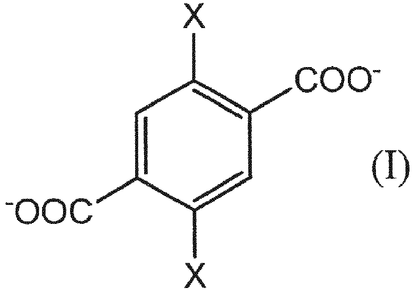 (wherein in formula (I), X is an unsubstituted or substituted C2-C20 alkyl group, an unsubstituted or substituted alkenyl group, an unsubstituted or substituted alkynyl group, an unsubstituted or substituted alkoxy group, an unsubstituted or substituted alkenyloxy group, an unsubstituted or substituted alkynyloxy group, a benzyloxy group, an unsubstituted or substituted alkylsulfanyl group, an unsubstituted or substituted alkenylsulfanyl group, an unsubstituted or substituted alkynylsulfanyl group, an unsubstituted or substituted alkylamino group, an unsubstituted or substituted dialkylamino group, an unsubstituted or substituted alkenylamino group, an unsubstituted or substituted dialkenylamino group, an unsubstituted or substituted alkynylamino group, an unsubstituted or substituted dialkynylamino group, a phenyl group, a sulfanyl group or an unsubstituted or substituted alkoxycarbonyl group).
[3] The hydrogen storage material according to [1] or [2], wherein the multivalent metal ion is an ion of at least one metal selected from the group consisting of Groups 2 to 13 metals in the periodic table of elements.
[4] The hydrogen storage material according to any one of [1] to [3], wherein the multivalent metal ion is an ion of at least one metal selected from Zn, Fe, Co, Ni, Cu, Al, Zr and Mg.
[5] A method for storing a hydrogen gas, comprising a step of contacting a hydrogen-containing gas with the hydrogen storage material according to any one of [1] to [4] to cause the hydrogen gas to be adsorbed inside the hydrogen storage material.
[6] A hydrogen storage tank filled with the hydrogen storage material according to any one of [1] to [4] or provided by forming the hydrogen storage material according to any one of [1] to [4].

### Effect of the Invention

The hydrogen storage material of the present invention is a novel hydrogen storage material. Use of this material enables hydrogen to be stored significantly than before.

### Mode of Carrying Out the Invention

The hydrogen storage material of the present invention contains an MOF comprising a carboxylate ion of formula (I) and a multivalent metal ion bound to each other; an MOF comprising an organic ligand containing a carboxylate ion of formula (I) and two or more nitrogen atoms (except for the carboxylate ion of formula (I)) and a multivalent metal ion bound to each other; or an MOF comprising a carboxylate ion of formula (I) and an organic ligand containing a nitrogen atom and a carboxy ionic group (except for the carboxylate ion of formula (I)) bound to a multivalent metal ion bound.

The multivalent metal ion used in the present invention is not particularly limited as long as the multivalent metal ion is a divalent or higher metal ion. The multivalent metal ion is preferably an ion of at least one metal selected from the group consisting of Groups 2 to 13 metals in the periodic table of elements, further, preferably an ion of at least one metal ion selected from Zn, Fe, Co, Ni, Cu, Al, Zr and Mg, and further preferably an ion of at least one metal selected from Co, Ni, Cu and Zn. These may be used singly or in combination of two or more.

These multivalent metal ions are provided in the form of various salts. Specific examples thereof can include Zn(NO₃)₂•6H₂O, Zn(NO₃)₂•4H₂O, Ni(NO₃)₂•6H₂O, Mg(NO₃)₂•6H₂O, Cu(NO₃)₂•xH₂O, Cu(NO₃)₂•2.5H₂O, Co(NO₃)₂•6H₂O, Al(NO₃)₃•6H₂O, ZrOCl₂•8H₂O and ZrCl₄. Further, in consideration of the purity of the salt, ease of bonding between the metal ion and the organic ligand and the like, nitrates are preferred.

The MOF used in the present invention comprises a carboxylate ion of formula (I).

In formula (I), X is an unsubstituted or substituted C2-C20 alkyl group, an unsubstituted or substituted alkenyl group, an unsubstituted or substituted alkynyl group, an unsubstituted or substituted alkoxy group, an unsubstituted or substituted alkenyloxy group, an unsubstituted or substituted alkynyloxy group, a benzyloxy group, an unsubstituted or substituted alkylsulfanyl group, an unsubstituted or substituted alkenylsulfanyl group, an unsubstituted or substituted alkynylsulfanyl group, an unsubstituted or substituted alkylamino group, an unsubstituted or substituted dialkylamino group, an unsubstituted or substituted alkenylamino group, an unsubstituted or substituted dialkenylamino group, an unsubstituted or substituted alkynylamino group, an unsubstituted or substituted dialkynylamino group, a phenyl group, a sulfanyl group or an unsubstituted or substituted alkoxycarbonyl group.

As used herein, the term "unsubstituted" means that only the group of a mother nucleus is present. When only the name of the group of a mother nucleus is described without the term "substituted", it means an "unsubstituted" group unless otherwise specified.

The term "substituted" means that any hydrogen atom of the group of a mother nucleus is replaced with a functional group (substituent) having the same structure as or different structure from the structure of the mother nucleus. Therefore, the "substituent" is another functional group attached to the functional group of a mother nucleus. The substituent may be one or more. The two or more substituents are the same as or different from each other.

Such terms as "C1-6" indicate that the number of carbon atoms of the group of a mother nucleus (the functional group following the term "C1 to 6") is 1 to 6, or the like. This number of carbon atoms does not include the number of carbon atoms present in the substituent. For example, a butyl group having an ethoxy group as a substituent is classified as a C2 alkoxy-C4 alkyl group.

The "substituent" is not particularly limited as long as it is chemically acceptable and has the effect of the present invention. Examples of the group that can be a "substituent" include:
a C1-6 alkyl group such as a methyl group, an ethyl group, an n-propyl group, an i-propyl group, an n-butyl group, an s-butyl group, an i-butyl group, a t-butyl group, an n-pentyl group or an n-hexyl group;
a C2-6 alkenyl group such as a vinyl group, a 1-propenyl group, a 2-propenyl group (allyl group), a propen-2-yl group, a 1-butenyl group, a 2-butenyl group, a 3-butenyl group, a 1-methyl-2-propenyl group or a 2-methyl-2-propenyl group;
a C2-6 alkynyl group such as an ethynyl group, a 1-propynyl group, a 2-propynyl group (propargyl group), a 1-butynyl group, a 2-butynyl group, a 3-butynyl group or a 1-methyl-2-propynyl group;
a C3-8 cycloalkyl group such as cyclopropyl group, a cyclobutyl group, a cyclopentyl group, a cyclohexyl group or cubanyl group;
a C6-10 aryl group such as a phenyl group or a naphthyl group;
a C6 to 10 aryl-Cl to 6 alkyl group such as a benzyl group or a phenethyl group;
a 3 to 6-membered heterocyclyl group;
a 3 to 6-membered heterocyclyl-Cl to 6 alkyl group;
an oxo group;
a hydroxyl group;
a C1-6 alkoxy group such as a methoxy group, an ethoxy group, an n-propoxy group, an i-propoxy group, an n-butoxy group, an s-butoxy group, an i-butoxy group or a t-butoxy group;
a C2-6 alkenyloxy group such as a vinyloxy group, an allyloxy group, a 1-propenyloxy group, a propen-2-yloxy group, a 3-butenyloxy group or a 2-butenyloxy group;
a C2-6 alkynyloxy group such as an ethynyloxy group or a propargyloxy group;
a C6-10 aryloxy group such as a phenoxy group or a naphthoxy group;
a C6-10 aryl-C1-6 alkoxy group such as a benzyloxy group or a phenethyloxy group;
a 5 to 6-membered heteroaryloxy group such as a thiazolyloxy group or a pyridyloxy group;
a 5 to 6-membered heteroaryl-C1-6 alkyloxy group such as a thiazolylmethyloxy group or a pyridylmethyloxy group;
a formyl group;
a C1-6 alkylcarbonyl group such as an acetyl group or a propionyl group;
a formyloxy group;
a C1-6 alkylcarbonyloxy group such as an acetyloxy group and a propionyloxy group;
a C6-10 arylcarbonyl group such as a benzoyl group;
a C1-6 alkoxycarbonyl group such as a methoxycarbonyl group, an ethoxycarbonyl group, an n-propoxycarbonyl group, an i-propoxycarbonyl group, an n-butoxycarbonyl group or a t-butoxycarbonyl group;
a C1-6 alkoxycarbonyloxy group such as a methoxycarbonyloxy group, an ethoxycarbonyloxy group, an n-propoxycarbonyloxy group, an i-propoxycarbonyloxy group, an n-butoxycarbonyloxy group or a t-butoxycarbonyloxy group;
a carboxy group;
a halogeno group such as a fluoro group, a chloro group, a bromo group or iodo group;
a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group or a 2,4,6-trichlorohexyl group;
a C2-6 haloalkenyl group such as a 2-chloro-1-propenyl group or a 2-fluoro-1-butenyl group;
a C2-6 haloalkynyl group such as a 4,4-dichloro-1-butynyl group, a 4-fluoro-1-pentynyl group or a 5-bromo-2-pentynyl group;
a C1-6 haloalkoxy group such as a trifluoromethoxy group, a 2-chloro-n-propoxy group or a 2,3-dichlorobutoxy group;
a C2-6 haloalkenyloxy group such as a 2-chloropropenyloxy group or a 3-bromobutenyloxy group;
a C1-6 haloalkylcarbonyl group such as a chloroacetyl group, a trifluoroacetyl group or a trichloroacetyl group;
an amino group;
a C1-6 alkyl-substituted amino group such as a methylamino group, dimethylamino group or a diethylamino group;
a C6-10 arylamino group such as an anilino group or a naphthylamino group;
a C6-10 aryl-C1-6 alkylamino group such as a benzylamino group or a phenethylamino group;
a formylamino group;
a C1-6 alkylcarbonylamino group such as an acetylamino group, a propanoylamino group, a butyrylamino group or an i-propylcarbonylamino group;
a C1-6 alkoxycarbonylamino group such as a methoxycarbonylamino group, an ethoxycarbonylamino group, an n-propoxycarbonylamino group or an i-propoxycarbonylamino group;
a C1-6 alkylsulfoxyimino group such as a S,S-dimethylsulfoxyimino group;
an unsubstituted aminocarbonyl group or an aminocarbonyl group having a substituent, such as an aminocarbonyl group, a dimethylaminocarbonyl group, a phenylaminocarbonyl group or an N-phenyl-N-methylaminocarbonyl group;
an imino-C1-6 alkyl group such as an iminomethyl group, a 1-iminoethyl group or a 1-imino-n-propyl group;
a substituted or unsubstituted N-hydroxyimino-C1-6 alkyl group such as an N-hydroxy-iminomethyl group, a 1-(N-hydroxyimino)ethyl group, a 1-(N-hydroxyimino)propyl group, an N-methoxyiminomethyl group or a 1-(N-methoxyimino)ethyl group;
a hydroxyimino group;
a C1-6 alkoxyimino group such as a methoxyimino group, an ethoxyimino group, an n-propoxyimino group, an i-propoxyimino group or an n-butoxyimino group;
an aminocarbonyloxy group;
a C1-6 alkyl-substituted aminocarbonyloxy group such as an ethylaminocarbonyloxy group or a dimethylaminocarbonyloxy group;
a thioxo group;
a sulfanyl group;
a C1-6 alkylsulfanyl group such as a methylsulfanyl group, an ethylsulfanyl group, an n-propylsulfanyl group, an i-propylsulfanyl group, an n-butylsulfanyl group, an i-butylsulfanyl group, an s-butylsulfanyl group and t-butylsulfanyl group;
a C1-6 haloalkylsulfanyl group such as a trifluoromethylsulfanyl group and a 2,2,2-trifluoroethylsulfanyl group;
a C6-10 arylsulfanyl group such as a phenylsulfanyl group and a naphthylsulfanyl group;
a 5 to 6-membered heteroarylsulfanyl group such as a thiazolylsulfanyl group and a pyridylsulfanyl group;
a C1-6 alkylsulfinyl group such as a methylsulfinyl group, an ethylsulfinyl group or a t-butylsulfinyl group;
a C1-6 haloalkylsulfinyl group such as a trifluoromethylsulfinyl group or a 2,2,2-trifluoroethylsulfinyl group;
a C6-10 arylsulfinyl group such as a phenylsulfinyl group;
a 5 to 6-membered heteroarylsulfinyl group such as a thiazolylsulfinyl group or a pyridylsulfinyl group;
a C1-6 alkylsulfonyl group such as a methylsulfonyl group, an ethylsulfonyl group or a t-butylsulfonyl group;
a C1-6 haloalkylsulfonyl group such as a trifluoromethylsulfonyl group or a 2,2,2-trifluoroethylsulfonyl group;
a C6-10 arylsulfonyl group such as a phenylsulfonyl group;
a 5 to 6-membered heteroarylsulfonyl group such as a thiazolylsulfonyl group or a pyridylsulfonyl group;
a sulfo group;
a C1-6 alkylsulfonyloxy group such as a methylsulfonyloxy group, an ethylsulfonyloxy group or a t-butylsulfonyloxy group;
a C1-6 haloalkylsulfonyloxy group such as a trifluoromethylsulfonyloxy group or a 2,2,2-trifluoroethylsulfonyloxy group;
a tri-C1-6 alkyl-substituted silyl group such as a trimethylsilyl group, a triethylsilyl group or a t-butyldimethylsilyl group;
a tri-C6-10 aryl-substituted silyl group such as a triphenylsilyl group;
a C2-C6 alkenyl-C1-C6 dialkyl-substituted silyl group such as an allyldimethylsilyl group or a vinyldimethylsilyl group;
a C1-C6 alkyl-di-C6-C10 aryl-substituted silyl group such as a t-butyldiphenylsilyl group or a diphenylmethylsilyl group;
a di-C1-C6 alkyl-C6-C10 aryl-substituted silyl group such as a dimethylphenylsilyl group;
a (C6-C10 phenyl-C1-C6 alkyl)-di-C1-C6 alkylsilyl group such a benzyldimethylsilyl group or a 3-phenylpropyldimethylsilyl group;
a C1-C6 alkyl-C6-C10 phenyl-C2-C6 alkenylsilyl group such as a methylphenylvinylsilyl group;
a tri-C1-C6 alkoxy-substituted silyl group such as a trimethoxysilyl group or a triethoxysilyl group;
a di-C1-C6 alkyl-substituted silyl group such as a dimethylsilyl group or a diethylsilyl group;
a di-C1-C6 alkoxy-substituted silyl group such as a dimethoxysilyl group or a diethoxysilyl group;
a C1-C6 alkoxy-C1-C6 alkyl-substituted silyl group such as a methoxydimethylsilyl group;
a C1-C6 alkoxy-C6-C10 aryl-substituted silyl group such as a t-butoxydiphenylsilyl group;
a C1-C6 alkyl-di-C1-C6 alkoxy-substituted silyl group such as a methyldimethoxysilyl group;
a cyano group; and
a nitro group.

In each of these "substituents", any hydrogen atom in the substituent may be also substituted with a group having a different structure. Such examples of the "substituent" can include a C1-6 alkyl group, a C1-6 haloalkyl group, a C1-6 alkoxy group, a C1-6 haloalkoxy group, a halogeno group, a cyano group or a nitro group.

The above-described "3 to 6-membered heterocyclyl group" contains, as a constituent atom of a ring, 1 to 4 heteroatoms selected from the group consisting of a nitrogen atom, an oxygen atom and a sulfur atom. The heterocyclyl group may be either monocyclic or polycyclic. As long as at least one ring in the polycyclic heterocyclyl group is a heterocyclic ring, the other ring(s) in the polycyclic heterocyclyl group may be any of a saturated alicyclic ring, an unsaturated alicyclic ring or an aromatic ring. Examples of the "3 to 6-membered heterocyclyl group" can include a 3 to 6-membered saturated heterocyclyl group, a 5 to 6-membered heteroaryl group and a 5 to 6-membered partially unsaturated heterocyclyl group.

Examples of the 3 to 6-membered saturated heterocyclyl group can include an aziridinyl group, an epoxy group, a pyrrolidinyl group, a tetrahydrofuranyl group, a thiazolidinyl group, a piperidyl group, a piperazinyl group, a morpholinyl group, a dioxolanyl group and a dioxanyl group.

Examples of the 5-membered heteroaryl group can include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group can include a pyridyl group, a pyrazinyl group, a pyrimidinyl group, a pyridazinyl group and a triazinyl group.

Examples of the 5 to 6-membered partially unsaturated heterocyclyl group can include an isoxazolinyl group and a pyrazolyl group.

Examples of the 3 to 6-membered heterocyclyl-1 to 6 alkyl group can include a glycidyl group, a 2-tetrahydrofuranylmethyl group, a 2-pyrrolylmethyl group, a 2-imidazolylmethyl group, a 3-isoxazolylmethyl group, a 5-isoxazolylmethyl group, a 2-pyridylmethyl group, a 4-pyridylmethyl group and a 3-isoxazolinylmethyl group.

In X in formula (I), the "alkyl group" of the "unsubstituted or substituted C2-C20 alkyl group" may be linear or branched, and examples thereof include an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, an i-hexyl group, a stearyl group, and a palmityl group.

In X in formula (I), examples of the "alkenyl group" of the "unsubstituted or substituted alkenyl group" can include a vinyl group, a 1-propenyl group, a 2-propenyl group (allyl group), a 1-butenyl group, a 2-butenyl group, a 3-butenyl group, a 1-methyl-2-propenyl group, a 2-methyl-2-propenyl group, butane-1,3-dien-1-yl, butane-1,3-dien-2-yl, a 1,2-propadien-1-yl group, a 1,1-dimethylallyl group, an oleyl group, a linoleyl group and a linolenyl group.

In X in formula (I), examples of the "alkynyl group" of the "unsubstituted or substituted alkynyl group" can include an ethynyl group, a 1-propynyl group, a 2-propynyl group (propargyl group), a 1-butynyl group, a 2-butynyl group, a 3-butynyl group, a 1-methyl-2-propynyl group and a 1,1-dimethylpropargyl group.

In X in formula (I), examples of the "alkoxy group" of the "unsubstituted or substituted alkoxy group" can include a methoxy group, an ethoxy group, an n-propoxy group, an i-propoxy group, an n-butoxy group, an s-butoxy group, an i-butoxy group, a t-butoxy group, an n-octyloxy group, a stearyloxy group and a palmityloxy group.

In X in formula (I), examples of the "alkenyloxy group" of the "unsubstituted or substituted alkenyloxy group" can include a vinyloxy group, an allyloxy group, a 1-propenyloxy group, a propen-2-yloxy group, a 3-butenyloxy group, a 2-butenyloxy group, a butane-1,3-diene-2-yloxy group, a 1,1-dimethylallyloxy group, an oleyloxy group, a linoleyloxy group and a linolenyloxy group.

In X in formula (I), examples of the "alkynyloxy group" of the "unsubstituted or substituted alkynyloxy group" can include an ethynyloxy group, a propargyloxy group, a 1-propyloxy group, a 3-butynyloxy group and 1,1-dimethylpropargyloxy group.

In X in formula (I), examples of the "alkylsulfanyl group" of the "unsubstituted or substituted alkylsulfanyl group" can include a methylsulfanyl group, an ethylsulfanyl group, an n-propylsulfanyl group, an i-propylsulfanyl group, an n-butylsulfanyl group, an i-butylsulfanyl group, an s-butylsulfanyl group, a t-butylsulfanyl group, a stearylsulfanyl group and a palmitylsulfanyl group.

In X in formula (I), examples of the "alkenylsulfanyl group" of the "unsubstituted or substituted alkenylsulfanyl group" can include a vinylsulfanyl group, an allylsulfanyl group, a 1-propenylsulfanyl group, a propen-2-ylsulfanyl group, a 3-butenylsulfanyl group, a 2-butenylsulfanyl group, a butane-1,3-dien-2-ylsulfanyl group, a 1,1-dimethylallylsulfanyl group, an oleylsulfanyl group, a linoleylsulfanyl group and a linolenylsulfanyl group.

In X in formula (I), examples of the "alkynylsulfanyl group" of the "unsubstituted or substituted alkynylsulfanyl group" can include an ethynylsulfanyl group, a propargylsulfanyl group, a 1-propylsulfanyl group, a 3-butynylsulfanyl group and a 1,1-dimethylpropargylsulfanyl group.

In X in formula (I), examples of the "alkylamino group" of the "unsubstituted or substituted alkylamino group" can include a methylamino group, an ethylamino group, an n-propylamino group, an isopropylamino group, an n-butylamino group, an s-butylamino group, a t-butylamino group, an isobutylamino group, a stearylamino group and a palmitylamino group.

In X in formula (I), examples of the "dialkylamino group" of the "unsubstituted or substituted dialkylamino group" can include a dimethylamino group, a diethylamino group, a di(n-propyl)amino group, a di(isopropyl)amino group, a di(n-butyl)amino group, a di(s-butylamino) group, a di(t-butylamino) group, a diisobutylamino group, a distearylamino group, a dipalmitylamino group, an ethylmethylamino group and an ethylisopropylamino group.

In X in formula (I), examples of the "alkenylamino group" of the "unsubstituted or substituted alkenylamino group" can include a vinylamino group, an allylamino group, a 1-propenylamino group, a 1-butenylamino group, a 2-butenylamino group, a 3-butenylamino group, a 2-methylallylamino group, a 2-methyl-1-propenylamino group, a 1,1-dimethylallylamino group, an oleylamino group, a linoleylamino group and a linolenylamino group.

In X in formula (I), examples of the "dialkenylamino group" of the "unsubstituted or substituted dialkenylamino group" can include a divinylamino group, a diallylamino group, a di(1-propenyl)amino group, a di(1-butenyl)amino group, a di(2-butenyl)amino group, a di(3-butenyl)amino group, a di(2-methylallyl)amino group, a bis(2-methyl-1-propenyl)amino group, a bis(1,1-dimethylallyl) group, a dioleylamino group, a dilinoleylamino group and a dilinolenylamino group.

In X in formula (I), examples of the "alkynylamino group" of the "unsubstituted or substituted alkynylamino group" can include an ethynylamino group, a propargylamino group, a 1-propynylamino group, a 1-butynylamino group, a 2-butynylamino group, a 3-butynylamino group and a 1,1-dimethylpropargylamino group.

In X in formula (I), examples of the "dialkynylamino group" of the "unsubstituted or substituted dialkynylamino group" can include a diethynylamino group, a dipropargylamino group, a di(1-propynyl)amino group, a di(1-butynyl)amino group, a di(2-butynyl)amino group, a di(3-butynyl)amino group and a bis(1,1-dimethylpropargyl)amino group.

In X in formula (I), examples of the "alkoxycarbonyl group" of the "unsubstituted or substituted alkoxycarbonyl group" can include a methoxycarbonyl group, an ethoxycarbonyl group, an n-propoxycarbonyl group, an i-propoxycarbonyl group, an n-butoxycarbonyl group, an s-butoxycarbonyl group, an i-butoxycarbonyl group, a t-butoxycarbonyl group, an n-octyloxycarbonyl group, an n-stearyloxycarbonyl group and a palmityloxycarbonyl group.

Examples of the carboxylate ion of formula (I) can include the following compounds:

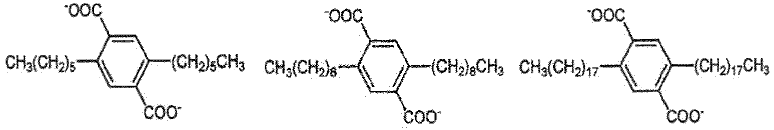

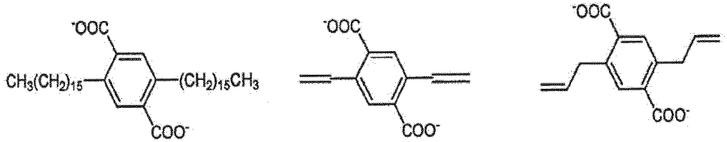

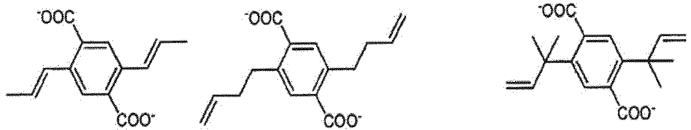

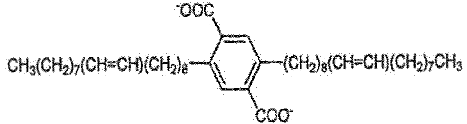

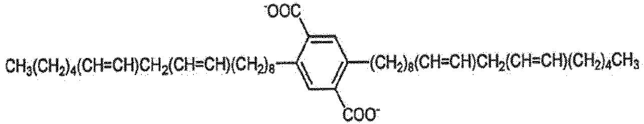

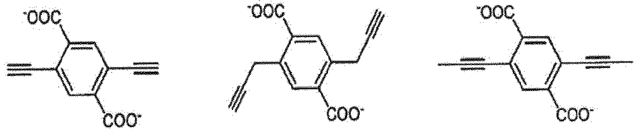

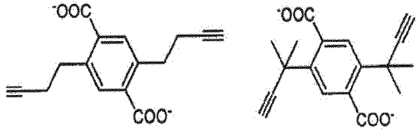

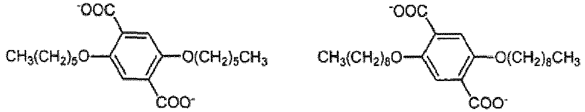

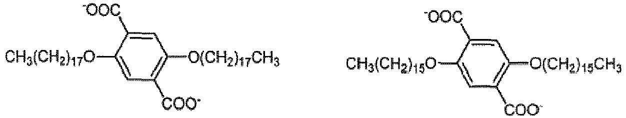

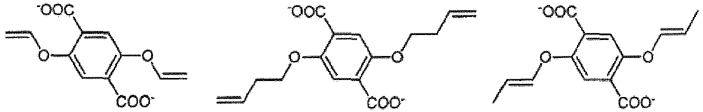

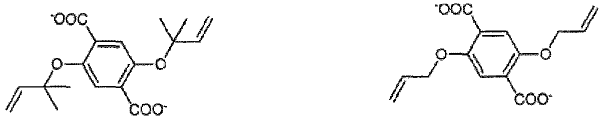

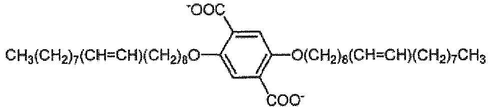

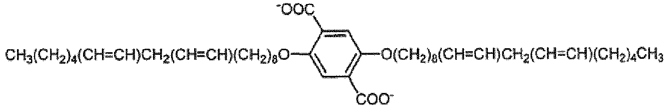

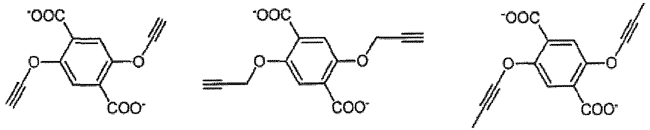

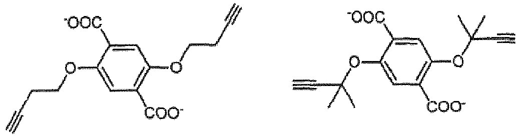

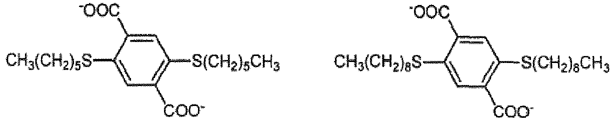

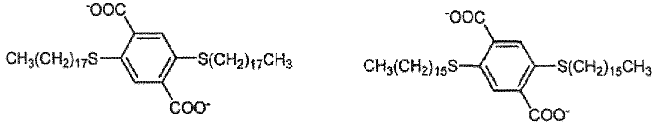

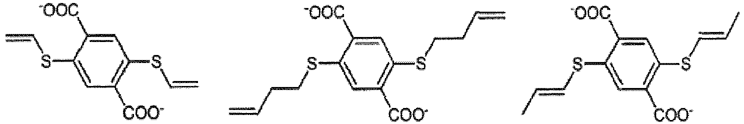

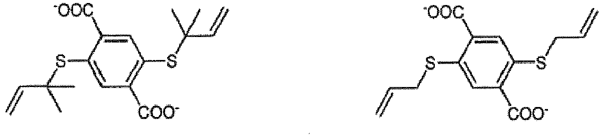

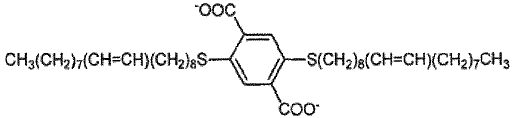

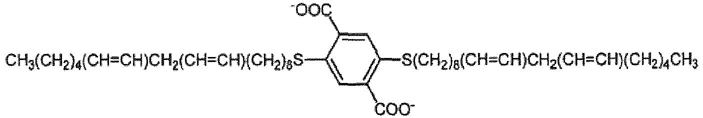

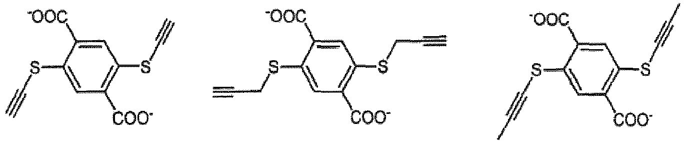

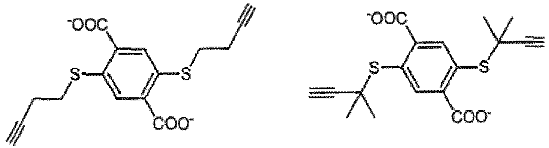

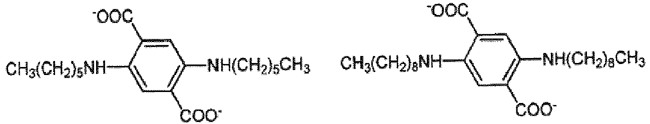

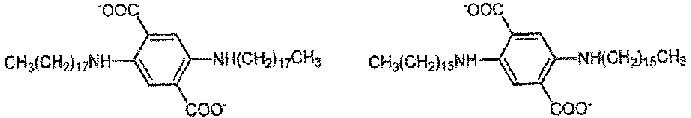

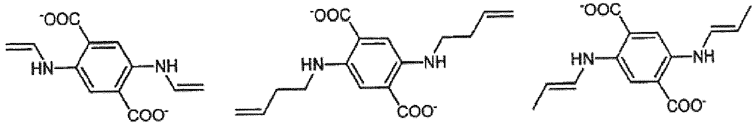

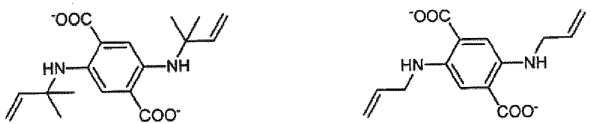

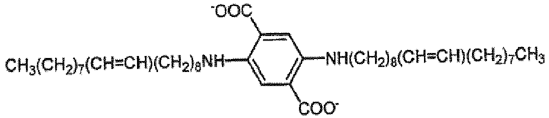

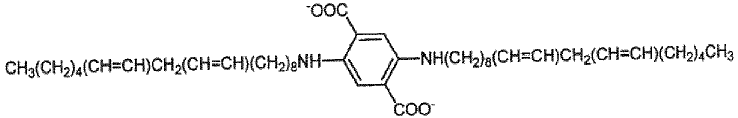

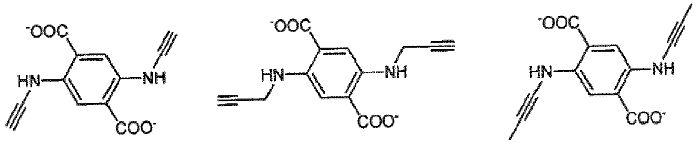

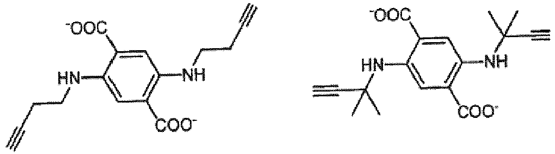

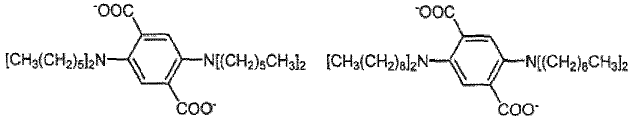

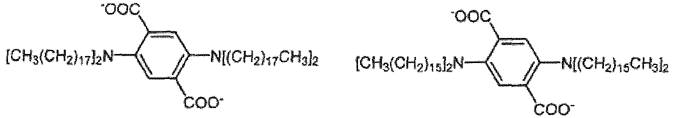

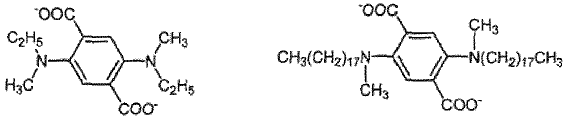

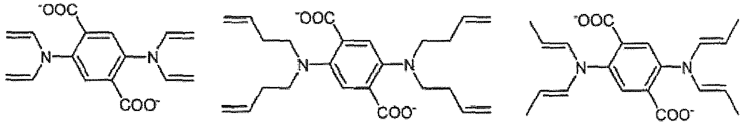

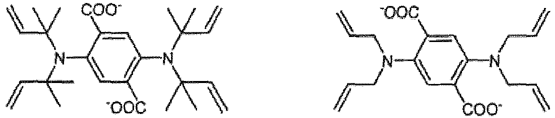

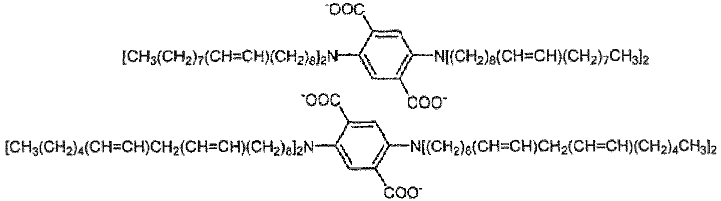

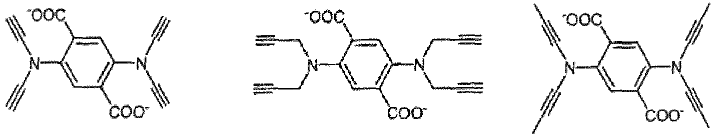

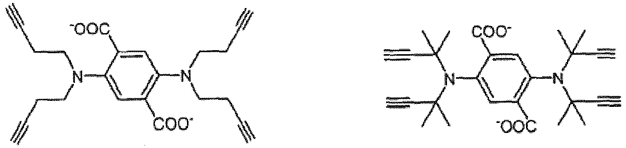

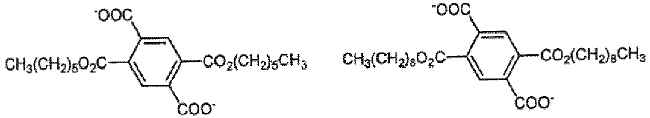

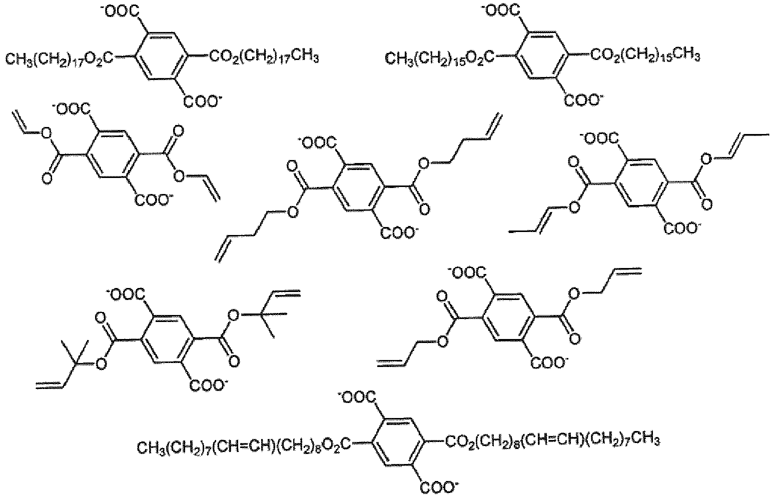

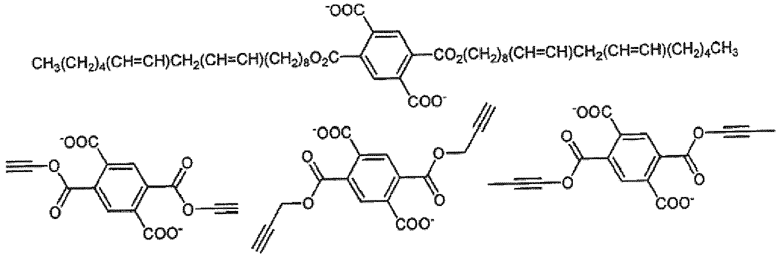

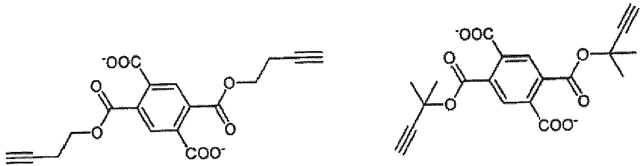

The hydrogen storage material of the present invention contains a metal-organic framework comprising a carboxylate ion of formula (I), a multivalent metal ion and an organic ligand containing two or more nitrogen atoms (except for the carboxylate ion of formula (I)) or an organic ligand containing a nitrogen atom and a carboxy ionic group (except for the carboxylate ion of formula (I)), wherein the carboxylate ion of formula (I) and the organic ligand are bound to the multivalent metal ion.

Examples of the organic ligand containing two or more nitrogen atoms used in the hydrogen storage material of the present invention can include benzimidazole, imidazole, 1,4-diazabicyclo[2.2.2]octane (DABCO), pyrazine, 4,4'-dipyridyl, 1,2-di(4-pyridyl)ethylene, 2,7-diazapyrene, 4,4'-azobispyridine and bis(3-(4-pyridyl)-2,4-pentanedionato)copper.

As for the organic ligand containing a nitrogen atom and a carboxy ionic group, examples of a carboxylic acid as the precursor thereof can include 4-pyridylcarboxylic acid, 4-pyridazinecarboxylic acid and 4-pyrimidinecarboxylic acid.

When the organic ligand of formula (I) and the organic ligand other than one of formula (I) are mixed and used, the mixing molar ratio is not particularly limited. However, in the case of a pillar molecule such as a pillar molecule that causes crosslinking to construct a three-dimensional structure such as a pillared-layer type structure, the organic ligand other than one of formula (I) is preferably used in an excessive amount relative to the organic ligand of formula (I).

Examples of the method for producing a metal-organic framework of the present invention that can be used include, but not limited to: a solution method such as a solvent diffusion method, a solvent agitation method or a hydrothermal method; a microwave method in which a reaction solution is irradiated with microwaves to uniformly heat the entire system in a short time; an ultrasonic method, in which a reaction vessel is irradiated with ultrasonic waves to repeatedly cause a change in pressure in the reaction vessel, leading to a phenomenon, referred to as cavitation, that a solvent forms bubbles and they collapse and in which a high energy field of about 5,000 K and 10,000 bar is locally formed and becomes a reaction field for nucleation of crystal; a solid-phase synthesis method in which a metal ion source and an organic ligand are mixed without using any solvent; and a liquid assisted grinding (LAG) method in which a metal ion source is mixed with an organic ligand with water added in an amount comparable to water of crystallization.

The method for producing a metal-organic framework of the present invention comprises, for example, steps of preparing a first solution containing a metal compound as a metal ion source and a solvent, a second solution containing a carboxylic acid as a precursor of the organic ligand of formula (I) and a solvent, and optionally, a third solution containing a compound to be another multidentate ligand and a solvent, respectively, and a step of mixing the first solution, the second solution and the third solution to prepare a reaction liquid and heating it to obtain a metal-organic framework. The first to third solutions do not need to be prepared separately. For example, the above metal compound, the carboxylic acid as the precursor of the organic ligand of formula (I), the compound to be another multidentate ligand and the solvent may be mixed at once to prepare one solution.

The mixing molar ratio of the above metal compound and the organic ligand of formula (I) can be any ratio selected depending on the pore size and surface characteristics of the metal-organic framework to be obtained, but the metal compound is preferably used in an amount of 1 mol or more and preferably used in an amount of further 1.1 mol or more, further 1.2 mol or more, further 1.5 mol or more, further 2 mol or more and further 3 mol or more relative to the organic ligand of formula (I).

The concentration of the above metal ion in the reaction liquid is preferably in the range of 25 to 200 mmol/L.

The concentration of the organic ligand of formula (1) in the reaction liquid is preferably in the range of 10 to 100 mol/L.

The concentration of the organic ligand other than the organic ligand of formula (I) in the reaction liquid is preferably in the range of 25 to 100 mol/L.

The solvent to be used can be one or more solvent selected from the group consisting of N, N-dimethylformamide (hereinafter may be described as "DMF"), N,N-diethylformamide (hereinafter may be described as "DEF"), N,N-dimethylacetamide and water. Among them, preferred is the use of N,N-dimethylformamide, N,N-diethylformamide or dimethylacetamide alone or alternatively the use of an N,N-dimethylformamide/water-mixed solvent, an N,N-diethylformamide/water-mixed solvent or an N,N-dimethylacetamide/water-mixed solvent.

The heating temperature of the reaction liquid is not particularly limited and is preferably in the range of room temperature to 140°C.

The method for storing hydrogen using the hydrogen storage material of the present invention is, but not particularly limited to, a method of contacting the hydrogen storage material of the present invention with a hydrogen gas, and the contacting manner is not particularly limited. Examples of the method include: a method in which a tank is filled with the hydrogen storage material of the present invention to form a hydrogen storage tank and the hydrogen gas is allowed to flow into the tank; a method in which the hydrogen storage material of the present invention is supported on the surface constituting the inner wall of the tank to form a hydrogen storage tank and the hydrogen gas is allowed to flow into the tank; and a method in which a tank is formed of the hydrogen storage material of the present invention as a hydrogen storage tank and the hydrogen gas is allowed to flow into the tank.

The hydrogen storage tank of the present invention can be configured by hermetically forming a material that may withstand a normal pressure or high pressure into a tank such that the tank has a space capable of storing hydrogen and filling the formed tank with the hydrogen storage material of the present invention.

Another aspect of the hydrogen storage tank of the present invention can be configured by mixing the hydrogen storage material of the present invention with another formable material as required and hermetically forming the mixed material into a tank in a form having a space inside of which hydrogen is allowed to flow. These aspects may configure, singly or in combination, the hydrogen storage tank of the present invention.

### Examples

Hereinafter, the present invention will be described in more detail with reference to Examples, but the present invention is not limited thereto.

The structures of carboxylic acids as the precursors of Organic ligands 1 to 14, of formula (I), used in the examples (hereinafter referred to as "Organic ligands" including the carboxylic acids as the precursors) are shown in Table 1.

Abbreviations in Table 1 denote the following meaning.

n-Pr: n-propyl group, i-Pr: isopropyl group, n-Bu: n-butyl group, Ph: phenyl group, Bn: benzyl group, Me: methyl group, Et: ethyl group, O: oxygen atom, N: nitrogen atom, H: hydrogen atom, C: carbon atom.

**[Table 1]**

| 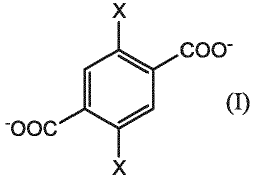 | | | |
|---|---|---|---|
| Organic ligand number | X | Organic ligand number | X |
| 1 | n-Pr | 8 | BnO |
| 2 | i-Pr | 9 | n-BuNH |
| 3 | n-Bu | 10 | (n-Bu)₂NCH₂ |
| 4 | Ph | 11 | MeOC(=O) |
| 5 | n-PrO | 12 | MeO |
| 6 | n-BuO | 13 | EtO |
| 7 | i-PrO | 14 | Et |

### [Production Example 1] Synthesis of Organic ligand 3

Iodine (2.6 mmol) was added to p-di(n-butyl)benzene (26.3 mmol), and the resultant was dissolved in chloroform. Bromine (52.6 mmol) was added dropwise thereto, and the resultant was stirred for 48 hours. 20% sodium hydroxide was added thereto, and the resultant was extracted with diethyl ether. The extracted solution was dried over magnesium sulfate and filtered. The filtrate was distilled under reduced pressure to obtain 2,5-dibromo-1,4-di(n-butyl)benzene (22.8 mmol). Copper cyanide (30 mmol) was added to the obtained 2,5-dibromo-1,4-di(n-butyl)benzene (10 mmol), and the resultant was dissolved in dimethylformamide (30 mL). The solution was refluxed overnight and returned to the room temperature. Then, aqueous ammonia (100 mL) was added thereto. The precipitated solid was filtered off, and the residue was washed sufficiently with water and dissolved in chloroform. After dried over magnesium sulfate, the solution was filtered, and the filtrate was distilled under reduced pressure. The obtained crude product was recrystallized in hexane to obtain 2,5-dicyano-1,4-di(n-butyl)benzene (9.5 mmol) as a colorless solid. A 10 M sodium hydroxide aqueous solution (75 mmol) was added to the obtained 2,5-dicyano-1,4-di(n-butyl)benzene (5.0 mmol), and the resultant was dissolved in ethylene glycol (50 mL). Then the solution was refluxed overnight. After it was returned to the room temperature, water (100 mL) was added thereto, and hydrochloric acid was added thereto to adjust the pH to 1. The precipitated solid was filtered off and dried sufficiently to obtain 1.3 g (4.7 mmol) of Organic ligand 3.

### [Production Example 2] Synthesis of Organic ligand 10

N-Bromosuccinimide (103 mmol) and azobisisobutyronitrile (1.72 mmol) were added to a solution of diethyl 2,5-dimethylterephthalate (42.9 mmol) in carbon tetrachloride (260 mL) and heated under reflux for 1 hour. Thereafter, the solution was subjected to hot filtration, and the filtrate returned to room temperature was distilled under reduced pressure. The obtained solid was washed with hexane to obtain diethyl 2,5-di(bromomethyl)terephthalate (34.1 mmol) as a crude product. The obtained bromomethyl form (2.45 mmol), dibutylamine (4.9 mmol) and triethylamine (4.9 mmol) were stirred in dichloromethane to obtain diethyl 2,5-bis(N,N-dibutylamino)terephthalate (2.43 mmol). 2,5-Bis(N,N-dibutylamino)terephthalate was hydrolyzed with sodium hydroxide (9.8 mmol) in ethanol and then neutralized with hydrochloric acid to obtain Organic ligand 10.

### [Comparative Example 1]

Terephthalic acid in an amount of 166.7 mg was dissolved in 13 mL of DMF, and 0.28 mL of triethylamine was added thereto. 17 mL of a solution of 557.7 mg of zinc acetate dihydrate in DMF was added dropwise thereto. The resultant was stirred at room temperature for 2.5 hours, and the obtained solid was separated by centrifugation. The supernatant was removed, and the solid was immersed in DMF (20 mL) overnight. Thereafter, the supernatant was removed by centrifugation, and substitution was made using chloroform. The operation of immersing the solid obtained by centrifugation in chloroform (20 mL) and centrifuging the immersed solid again was repeated three times. Thereafter, the solid obtained by centrifugation was dried under vacuum at 150°C for 5 hours to obtain Metal-organic framework A (white powder, 142.9 mg).

### [Comparative Example 2]

DMF (50 mL) was added to 0.492 g of 2,5-dimethylterephthalic acid and 1.49 g of zinc nitrate hexahydrate and heated in an oven (reaction conditions: 120°C, 24 hours). It was returned to room temperature, and the supernatant was removed. DMF (50 mL) was added thereto, the supernatant was removed, and the solvent was replaced with chloroform. Chloroform (50 mL) was added thereto, and immersion was conducted overnight. The solid was subjected to suction filtration, and the obtained solid was dried under vacuum at 150°C for 5 hours to obtain Metal-organic framework B (0.709 g).

### [Production Example 1-1]

Organic ligand 4 (0.75 mmol), zinc nitrate hexahydrate (0.75 mmol), 1,4-diazabicyclo[2.2.2]octane (0.40 mmol) and DMF (15 mL) were stirred for 15 minutes at room temperature and under ultrasonication. Thereafter, the filtrate obtained by centrifugation and filtration was placed in an autoclave and heated in an oven (reaction conditions: 120°C, 48 hours). It was returned to room temperature, the supernatant was discarded, and then the crystal was washed with DMF. Thereafter, the crystal was immersed in DMF overnight. Thereafter, the supernatant was removed and replaced with chloroform. Then the crystal was immersed for 1 day. The step of removing the supernatant after centrifugation was repeated three times. The solid obtained by centrifugation and removal of the supernatant was dried under vacuum at 150°C to obtain Metal-organic framework 1-1 as the target compound.

### [Production Example 1-2]

Metal-organic framework 1-2 (white solid) was obtained by the same operation as in Production Example 1-1 except that Organic ligand 2 was used instead of Organic ligand 4.

### [Production Example 1-3]

Metal-organic framework 1-3 (white solid) was obtained by the same operation as in Production Example 1-1 except that Organic ligand 7 was used instead of Organic ligand 4.

### [Production Example 1-4]

Metal-organic framework 1-4 (colorless crystal) was obtained by the same operation as in Production Example 1-1 except that Organic ligand 13 was used instead of Organic ligand 4.

### [Production Example 2-1]

Organic ligand 4 (0.485 mmol) was dissolved in DMF (7 mL). A solution of zinc acetate dihydrate (1.29 mmol) in DMF (9 mL) was added dropwise thereto. The resultant was stirred at room temperature for 2.5 hours, and the obtained solid was separated by centrifugation. The supernatant was removed, and the solid was immersed in DMF (20 mL) overnight. Thereafter, the supernatant was removed by centrifugation, and substitution was made using chloroform. The washing operation of immersing the solid obtained by centrifugation in chloroform (20 mL) overnight and centrifuging the immersed solid again was repeated three times. Thereafter, the solid obtained by centrifugation was dried under vacuum at 150°C for 5 hours to obtain Metal-organic framework 2-1.

### [Production Example 2-2]

Metal-organic framework 2-2 (white powder) was obtained by the same operation as in Production Example 2-1 except that Organic ligand 7 was used instead of Organic ligand 4.

### [Production Example 2-3]

Metal-organic framework 2-3 (white powder) was obtained by the same operation as in Production Example 2-1 except that Organic ligand 2 was used instead of Organic ligand 4.

### [Production Example 2-4]

Metal-organic framework 2-4 (pale purple powder) was obtained by the same operation as in Production Example 2-1 except that Organic ligand 1 was used instead of Organic ligand 4.

### [Production Example 2-5]

Metal-organic framework 2-5 (colorless powder) was obtained by the same operation as in Production Example 2-1 except that Organic ligand 13 was used instead of Organic ligand 4.

### [Production Example 2-6]

Metal-organic framework 2-6 (colorless powder) was obtained by the same operation as in Production Example 2-1 except that Organic ligand 12 was used instead of Organic ligand 4.

### [Production Example 3-1]

Organic ligand 7 (1.0 mmol) was dissolved in DMF (13 mL), and triethylamine (0.28 mL) was added thereto. A solution of zinc acetate dihydrate (2.5 mmol) in DMF (17 mL) was added dropwise thereto. The resultant was stirred at room temperature for 2.5 hours, and the obtained solid was separated by centrifugation. The supernatant was removed, and the solid was immersed in DMF (20 mL) overnight. Thereafter, the supernatant was removed by centrifugation, and substitution was made using chloroform. The washing operation of immersing the solid obtained by centrifugation in chloroform (20 mL) overnight and centrifuging the immersed solid again was repeated three times. Thereafter, the solid obtained by centrifugation was dried under vacuum at 150°C for 5 hours to obtain Metal-organic framework 3-1 (white powder).

### [Production Example 3-2]

Metal-organic framework 3-2 (white powder) was obtained by the same operation as in Production Example 3-1 except that Organic ligand 2 was used instead of Organic ligand 7.

### [Production Example 3-3]

Metal-organic framework 3-3 (pale purple powder) was obtained by the same operation as in Production Example 3-1 except that Organic ligand 1 was used instead of Organic ligand 7.

### [Production Example 3-4]

Metal-organic framework 3-4 (colorless powder) was obtained by the same operation as in Production Example 3-1 except that Organic ligand 13 was used instead of Organic ligand 7.

### [Production Example 4-1]

DMF (40 mL) was added to Organic ligand 7 (1.2 mmol) and zinc nitrate hexahydrate (1.6 mmol) and stirred for 5 minutes. Triethylamine (14.4 mmol) was added dropwise thereto and stirred at room temperature for 15 minutes. After still standing for 20 minutes, the resultant was centrifuged for 10 minutes to remove the supernatant. DMF (10 mL) was added to the residue, and the resultant was centrifuged again for 10 minutes to remove the supernatant. After immersion in DMF overnight, the resultant was centrifuged for 10 minutes to remove the supernatant, and then chloroform (10 mL) was added thereto. The washing operation of immersing the solid obtained by centrifugation in chloroform (20 mL) overnight and centrifuging the immersed solid again was repeated three times. Thereafter, the solid obtained by centrifugation was dried under vacuum at 150°C for 5 hours to obtain Metal-organic framework 4-1.

### [Production Example 4-2] to [Production Example 4-9]

Metal-organic frameworks 4-2 to 4-9 were obtained by the same operation as in Production Example 4-1 except that Organic ligands shown in Table 2 were used instead of Organic ligand 7.

**[Table 2]**

| Production Example | Organic ligand | Property |
|---|---|---|
| 4-1 | 7 | |
| 4-2 | 8 | |
| 4-3*¹ | 5 | |
| 4-4 | 6 | |
| 4-5*² | 2 | White powder |
| 4-6*² | 1 | White powder |
| 4-7*² | 13 | Colorless powder |
| 4-8 | 14 | Colorless powder |
| 4-9 | 3 | Colorless powder |

| | | |
|---|---|---|
| *1: Stirred at 120°C for 24 hours with no triethylamine added. *2: Stirring time was set to 5 minutes. | | |

### [Production Example 5-1]

DMF (20 mL) was added to Organic ligand 7 (0.1 mmol) and zinc nitrate hexahydrate (0.3 mmol) and heated in an oven (reaction conditions: 120°C, 24 hours). It was returned to room temperature and centrifuged, and then the supernatant was removed. DMF (10 mL) was added thereto, the resultant was centrifuged, and then the solvent was removed and replaced with chloroform. Chloroform (10 mL) was added thereto, and immersion was conducted overnight. After removal of the chloroform by centrifugation, the residue was dried under vacuum at 150°C for 5 hours to obtain Metal-organic framework 5-1.

### [Production Example 5-2] to [Production Example 5-15]

Metal-organic frameworks 5-2 to 5-15 were obtained by the same operation as in Example 5-1 except that Organic ligands and solvents shown in Table 3 below were used and the reaction was conducted under the reaction conditions shown in Table 3. The results are shown in Table 3.

**[Table 3]**

| Production Example | Organic ligand | Solvent | Temperature (°C) | Heating time (hrs.) | Property |
|---|---|---|---|---|---|
| 5-1 | 7 | DMF | 120 | 24 | |
| 5-2 | 9 | DMF | 120 | 24 | |
| 5-3 | 10 | DMF | 120 | 24 | |
| 5-4 | 2 | DEF | 120 | 24 | |
| 5-5 | 1 | DMF | 120 | 24 | Colorless crystal |
| 5-6 | 1 | DEF | 120 | 24 | Colorless crystal |
| 5-7 | 1 | DMF | 90 | 24 | |
| 5-8 | 7 | DEF | 120 | 24 | Light gray yellow crystal |
| 5-9 | 7 | DEF | 90 | 48 | Light gray yellow crystal |
| 5-10 | 11 | DEF | 120 | 24 | Pale yellow crystal |
| 5-11 | 3 | DMF | 120 | 24 | Off-white crystal |
| 5-12 | 3 | DEF | 120 | 24 | Pale yellow crystal |
| 5-13 | 13 | DEF | 120 | 24 | |
| 5-14 | 14 | DMF | 120 | 48 | Pale orange crystal |
| 5-15 | 14 | DEF | 120 | 24 | Pale yellow crystal |

### [Production Example 6-1]

Organic ligand 1 (250 mg, 1.0 mmol), aluminum nitrate nonahydrate (750 mg, 2.0 mmol) and water (3 mL) were placed in an autoclave and heated at 200°C for 12 hours. It was returned to room temperature and centrifuged, and then the supernatant was removed. The operation of adding DMF (10 mL), centrifuging the resultant and removing the supernatant was repeated twice. Thereafter, the operation of adding water (10 mL), centrifuging the resultant and removing the supernatant in the same manner was repeated twice. The resultant was dried under vacuum at 150°C for 5 hours to obtain Metal-organic framework 6-1 (brown powder, 301 mg).

### [Production Example 7-1]

Organic ligand 1 (62.6 mg, 0.25 mmol) dissolved in ethanol (15 mL) was placed in an autoclave along with an aqueous solution (15 mL) of copper nitrate 2.5 hydrate (116 mg, 0.60 mmol) and heated at 140°C for 24 hours. It was returned to room temperature and centrifuged, and then the supernatant was removed. The operation of adding water (10 mL), centrifuging the resultant and removing the supernatant was repeated three times. Thereafter, the operation of adding ethanol (10 mL), centrifuging the resultant and removing the supernatant in the same manner was repeated three times. The resultant was dried under vacuum at 150°C for 5 hours to obtain Metal-organic framework 7-1 (blue powder, 47 mg).

### [Production Example 8-1]

DMF (20 mL) and ethanol (10 mL) were added to Organic ligand 4 (63.9 mg, 0.20 mmol), zinc nitrate hexahydrate (59.8 mg, 0.20 mmol) and 4,4'-bipyridine (31.7 mg, 0.20 mmol) and stirred at room temperature for 15 minutes. The suspension was filtered through Celite, and the obtained solution was placed in an autoclave and heated at 85°C for 48 hours. It was returned to room temperature, DMF was added thereto, and immersion was conducted overnight. Then the supernatant was removed, the solvent was replaced with chloroform, chloroform (10 mL) was added thereto, and immersion was conducted for 1 day. The operation of centrifugation, removal of the solvent, addition of chloroform again, immersion overnight, and removal of the supernatant after centrifugation was repeated three times. The solid obtained by centrifugation and removal of the supernatant was dried under vacuum at 150°C to obtain Metal-organic framework 8-1 (51.5 mg) as the target compound.

### [Production Example 9-1]

DMF (10 mL) and formic acid (1.8 mL) were added to Organic ligand 1 (63.2 mg, 0.25 mmol) and zirconium chloride oxide octahydrate (83.6 mg, 0.25 mmol) and heated at 120°C for 24 hours. It was returned to room temperature, DMF was added thereto, and immersion was conducted overnight. Then the supernatant was removed, the solvent was replaced with chloroform, chloroform (10 mL) was added thereto, and immersion was conducted for 1 day. The operation of centrifugation, removal of the solvent, addition of chloroform again, immersion overnight, and removal of the supernatant after centrifugation was repeated three times. The solid obtained by centrifugation and removal of the supernatant was dried under vacuum at 150°C to obtain Metal-organic framework 9-1 (47.1 mg) as the target compound.

### [Production Example 10-1]

THF in an amount of 9 mL and water in an amount of 1 mL were added to 0.102 g of Organic ligand 13 and 0.233 g of nickel nitrate hexahydrate and heated in an oven (reaction conditions: 100°C, 24 hours). It was returned to room temperature, centrifuged and decanted to obtain a solid. The operation of adding DMF to the solid and centrifuging and decanting it was repeated three times. Then the solid was immersed overnight. The operation of removing the supernatant, adding chloroform to the solid, and centrifuging and decanting it was repeated three times. The solvent was replaced with chloroform, and the solid was immersed overnight. The solid obtained by decanting the solid was dried under vacuum at 150°C for 5 hours to obtain 0.047 g of Metal-organic framework 10-1.

### [Production Example 10-2] to [Production Example 10-5]

Metal-organic frameworks 10-2 to 10-5 were obtained in the same manner as in Production Example 10-1 except that Organic ligands shown in Table 4 below were used and the reaction conditions shown in Table 4 were used. The results are shown in Table 4.

**[Table 4]**

| Production Example | Organic ligand | Heating time | |
|---|---|---|---|
| 10-1 | 13 | 24 | Lime green crystal |
| 10-2 | 7 | 48 | Bark solid |
| 10-3 | 3 | 48 | Yellow bark powder |
| 10-4 | 12 | 48 | Yellow bark solid |
| 10-5 | 2 | 48 | Yellow powder |

### [Production Example 11-1]

0.077 g of Organic ligand 13, 0.071 g of zirconium chloride, 0.065 g of water, 0.541 g of acetic acid and 4 mL of DMF were stirred for 5 minutes at room temperature and under ultrasonication. Thereafter, the mixture was heated in an oven (reaction conditions: 120°C, 24 hours). It was returned to room temperature, centrifuged and decanted to obtain a solid. The operation of adding DMF to the solid and centrifuging and decanting it was repeated three times. The supernatant was removed, and DMF was added thereto to immerse the solid overnight. The operation of decanting the immersed solid after centrifugation, adding acetone to the obtained solid, and centrifuging and decanting it was repeated three times. The supernatant was removed, and acetone was added thereto to immerse the solid overnight. The solid obtained by centrifugation and decantation was dried under vacuum at 150°C for 5 hours to obtain 0.053 g of Metal-organic framework 11-1.

### [Production Example 11-2] to [Production Example 11-5]

Metal-organic frameworks 11-2 to 11-5 were obtained in the same manner as in Production Example 11-1 except that Organic ligands shown in Table 5 below were used. The results are shown in Table 5.

**[Table 5]**

| | | |
|---|---|---|
| Production | Organic | Property |

| Example | ligand | |
|---|---|---|
| 11-1 | 13 | Colorless solid |
| 11-2 | 12 | Colorless powder |
| 11-3 | 2 | Beige solid |
| 11-4 | 7 | Pale green powder |
| 11-5 | 1 | Colorless powder |

### [Production Example 12-1]

Organic ligand 13 in an amount of 0.0510 g, 0.0489 g of copper nitrate trihydrate and 4 mL of DMF were added and stirred, and then the mixture was filtered. Thereafter, the mixture was heated in an oven (reaction conditions: 120°C, 24 hours). It was returned to room temperature, centrifuged and decanted to obtain a solid. The operation of adding DMF to the solid and centrifuging and decanting it was repeated three times. The supernatant was removed, and the solvent was replaced with chloroform. The operation of addition of chloroform, washing and filtration under pressure was repeated three times, and chloroform was added thereto to immerse the solid overnight. The solid was subjected to filtration under pressure, and the obtained solid was dried under vacuum at 150°C for 5 hours to obtain 0.0488 g of Metal-organic framework 12-1.

### [Production Example 12-2] to [Production Example 12-10]

Metal-organic frameworks 12-2 to 12-10 were obtained in the same manner as in Production Example 12-1 except that Organic ligands shown in Table 6 below were used and the reaction conditions shown in Table 6 were used. The results are shown in Table 6.

**[Table 6]**

| Production Example | Organic ligand | Heating time | Heating temperature | Solvent | Property |
|---|---|---|---|---|---|
| 12-1 | 13 | 24 | 120 | DMF | Green crystal |
| 12-2 | 13 | 24 | 90 | DMF | Green crystal |
| 12-3 | 13 | 24 | 120 | DEF | Green crystal |
| 12-4 | 13 | 24 | 90 | DEF | Green crystal |
| 12-5 | 7 | 24 | 120 | DMF | Emerald green crystal |
| 12-6 | 7 | 24 | 90 | DMF | Emerald green crystal |
| 12-7 | 7 | 24 | 120 | DEF | Emerald green crystal |
| 12-8 | 7 | 24 | 90 | DEF | Emerald green crystal |
| 12-9 | 3 | 43 | 120 | DMF | Light blue powder |
| 12-10 | 3 | 43 | 90 | DMF | Light blue powder |

### [Example 1]

### (Measurement of BET specific surface area and measurement of hydrogen storage capacity)

The BET specific surface area and the hydrogen storage capacity at 77 K and atmospheric pressure were measured for some of Metal-organic frameworks obtained.

The BET specific surface area and the hydrogen storage capacity at 77 K and atmospheric pressure were measured with a gas adsorption measurement device, Tristar-II (manufactured by Micromeritics Instrument Corporation)

The BET specific surface area was calculated according to the following procedure. About 50 mg of each of Metal-organic frameworks was placed inside a glass cell. The pressure inside the glass cell was reduced to vacuum at a temperature of 135°C and the inside of the glass cell was dried for 6 hours. The glass cell was attached to the gas adsorption measurement device and immersed in a temperature-controlled bath containing liquid nitrogen. The pressure of nitrogen contained in the glass cell was gradually increased. The measurement was carried out until the pressure of nitrogen introduced into the glass cell reached 1.0 × 10⁵ Pa.

The hydrogen storage capacity at 77K and a normal pressure was calculated according to the following procedure. After the measurement for nitrogen, the gas type was changed to hydrogen to carry out the measurement. The pressure of hydrogen contained in the glass cell was gradually increased. The measurement was carried out until the pressure of hydrogen introduced into the glass cell reached 1.0 × 10⁵ Pa.

The results of the BET specific surface areas measured were shown in Table 7.

The hydrogen storage capacities measured at 77 K and atmospheric pressure are shown in Table 8.

**[Table 7]**

| Metal-organic framework number | BET specific surface area (m²/g) | Metal-organic framework number | BET specific surface area (m²/g) |
|---|---|---|---|
| 2-2 | 1160 | 5-4 | 574 |
| 2-3 | 1688 | 5-5 | 1671 |
| 2-4 | 1470 | 5-6 | 1665 |
| 2-5 | 1389 | 5-7 | 1640 |
| 2-6 | 1194 | 5-8 | 1033 |
| 3-1 | 1319 | 5-9 | 1105 |
| 3-2 | 1007 | 5-11 | 1384 |
| 3-3 | 3789 | 5-12 | 1306 |
| 3-4 | 1082 | 5-14 | 2094 |
| 4-1 | 517 | 5-15 | 2056 |
| 4-5 | 1485 | 9-1 | 433 |
| 4-6 | 1738 | 11-1 | 444 |
| 4-7 | 555 | 11-2 | 698 |
| 4-8 | 1907 | 11-4 | 432 |
| 4-9 | 1035 | 11-5 | 508 |
| 5-1 | 518 | B | 426 |

**[Table 8]**

| Metal-organic framework number | Hydrogen storage capacity wt% | Metal-organic framework number | Hydrogen storage capacity wt% |
|---|---|---|---|
| 2-2 | 1.299 | 5-6 | 1.46 |
| 2-3 | 1.706 | 5-7 | 1.188 |
| 2-5 | 1.448 | 5-8 | 1.181 |
| 2-6 | 1.237 | 5-9 | 1.295 |
| 3-1 | 1.448 | 5-11 | 1.31 |
| 3-3 | 3.240 | 5-12 | 1.23 |
| 3-4 | 1.235 | 5-14 | 1.58 |
| 4-5 | 1.546 | 5-15 | 1.61 |
| 4-6 | 1.542 | 11-2 | 1.161 |
| 4-8 | 1.57 | A | 1.111 |
| 5-5 | 1.43 | | |

### Industrial Applicability

The hydrogen storage material of the present invention can store hydrogen at a practical level. Consequently, the metal-organic framework can make hydrogen to be utilized more easily, toward the advent of a hydrogen energy-based society.

## Claims

1. A hydrogen storage material containing a metal-organic framework, the metal-organic framework comprising a carboxylate ion of formula (I) and a multivalent metal ion, wherein the carboxylate ion and the multivalent metal ion are bound to each other: 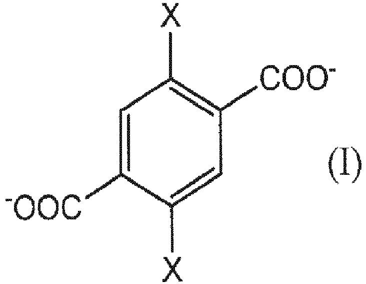 (wherein in formula (I), X is
an unsubstituted or substituted C2-C20 alkyl group,
an unsubstituted or substituted alkenyl group,
an unsubstituted or substituted alkynyl group,
an unsubstituted or substituted alkoxy group,
an unsubstituted or substituted alkenyloxy group,
an unsubstituted or substituted alkynyloxy group,
a benzyloxy group,
an unsubstituted or substituted alkylsulfanyl group,
an unsubstituted or substituted alkenylsulfanyl group,
an unsubstituted or substituted alkynylsulfanyl group,
an unsubstituted or substituted alkylamino group,
an unsubstituted or substituted dialkylamino group,
an unsubstituted or substituted alkenylamino group,
an unsubstituted or substituted dialkenylamino group,
an unsubstituted or substituted alkynylamino group,
an unsubstituted or substituted dialkynylamino group,
a phenyl group,
a sulfanyl group,
or
an unsubstituted or substituted alkoxycarbonyl group.)

2. A hydrogen storage material containing a metal-organic framework, the metal-organic framework comprising:
a carboxylate ion of formula (I);
a multivalent metal ion; and
an organic ligand containing two or more nitrogen atoms (with the proviso that the carboxylate ion of formula (I) is excluded) or an organic ligand containing a nitrogen atom and a carboxy ionic group (with the proviso that the carboxylate ion of formula (I) is excluded);
wherein the carboxylate ion of formula (I) and the organic ligand are bound to the multivalent metal ion: 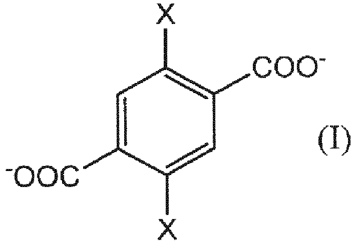 (wherein in formula (I), X is
an unsubstituted or substituted C2-C20 alkyl group,
an unsubstituted or substituted alkenyl group,
an unsubstituted or substituted alkynyl group,
an unsubstituted or substituted alkoxy group,
an unsubstituted or substituted alkenyloxy group,
an unsubstituted or substituted alkynyloxy group,
a benzyloxy group,
an unsubstituted or substituted alkylsulfanyl group,
an unsubstituted or substituted alkenylsulfanyl group,
an unsubstituted or substituted alkynylsulfanyl group,
an unsubstituted or substituted alkylamino group,
an unsubstituted or substituted dialkylamino group,
an unsubstituted or substituted alkenylamino group,
an unsubstituted or substituted dialkenylamino group,
an unsubstituted or substituted alkynylamino group,
an unsubstituted or substituted dialkynylamino group,
a phenyl group,
a sulfanyl group,
or
an unsubstituted or substituted alkoxycarbonyl group.)

3. The hydrogen storage material according to claim 1 or 2, wherein the multivalent metal ion is an ion of at least one metal selected from the group consisting of Groups 2 to 13 metals in the periodic table of elements.

4. The hydrogen storage material according to any one of claims 1 to 3, wherein the multivalent metal ion is an ion of at least one metal selected from Zn, Fe, Co, Ni, Cu, Al, Zr and Mg.

5. A method for storing a hydrogen gas, comprising a step of contacting a hydrogen-containing gas with the hydrogen storage material according to any one of claims 1 to 4 to cause the hydrogen gas to be adsorbed inside the hydrogen storage material.

6. A hydrogen storage tank filled with the hydrogen storage material according to any one of claims 1 to 4 or provided by forming the hydrogen storage material according to any one of claims 1 to 4.
